# EUROPEAN PATENT SPECIFICATION

(11) **EP 1 967 209 B1**
(45) Date of publication and mention of the grant of the patent: **06.06.2012**
(21) Application number: 06833196.6
(22) Date of filing: 24.11.2006
(51) Int. Cl.: A61K 45/00, A61K 39/395, A61P 13/08, A61P 35/00

(54) **THERAPEUTIC AGENT FOR PROSTATE CANCER**
THERAPEUTISCHES MITTEL GEGEN PROSTATAKREBS
AGENT THÉRAPEUTIQUE POUR LE CANCER DE LA PROSTATE

(30) Priority: 25.11.2005 JP 2005340050
(43) Date of publication of application: 10.09.2008
(73) Proprietor: Keio University, Tokyo 108-8345 (JP); CHUGAI SEIYAKU KABUSHIKI KAISHA, Tokyo, 115-8543 (JP)
(72) Inventor: NAKASHIMA, Jun, Tokyo 160-8582 (JP); KANAO, Kent, Tokyo 160-8582 (JP)
(74) Representative: Vossius & Partner
(86) International application number: PCT/JP2006/323392
(87) International publication number: WO 2007/061029

(56) References cited:
- WO-A1-2005/044848
- WO-A1-2005/107800
- WO-A2-2004/071404
- BORSELLINO N ET AL: "Blocking signaling through the Gp130 receptor chain by interleukin-6 and oncostatin M inhibits PC-3 cell growth and sensitizes the tumor cells to etoposide and cisplatin-mediated cytotoxicity." CANCER, vol. 85, no. 1, 1999, pages 134-144, XP002543275
- LEE SOO OK ET AL: "Interleukin-6 protects LNCaP cells from apoptosis induced by androgen deprivation through the Stat3 pathway." THE PROSTATE, vol. 60, no. 3, 2004, pages 178-186, XP002543276
- DAVIES GAYNOR ET AL: "The HGF/SF antagonist NK4 reverses fibroblast- and HGF-induced prostate tumor growth and angiogenesis in vivo." INTERNATIONAL JOURNAL OF CANCER, vol. 106, no. 3, 2003, pages 348-354, XP002543277
- XING Y; ET AL: "THE EFFECT OF INTERLEUKIN-6 ON THE PROLIFERATION OF PROSTATE CANCER CELLS IN VITRO AND THE MODULATION OF THIS PROCEDURE" JOURNAL OF TONGJI MEDICAL UNIVERSITY, vol. 21, no. 3, 2001, pages 225-227, XP008047142
- CULIG ZORAN ET AL: "Interleukin-6 regulates androgen receptor activity and prostate cancer cell growth." MOLECULAR AND CELLULAR ENDOCRINOLOGY, vol. 197, no. 1-2, 2002, pages 231-238, XP002543278
- EDER IRIS E ET AL: "Targeting the androgen receptor in hormone-refractory prostate cancer--new concepts." FUTURE ONCOLOGY, vol. 1, no. 1, February 2005 (2005-02), pages 93-101, XP002543279
- TRIKHA MOHIT ET AL: "Targeted anti-interleukin-6 monoclonal antibody therapy for cancer: a review of the rationale and clinical evidence." CLINICAL CANCER RESEARCH, vol. 9, no. 13, 2003, pages 4653-4665, XP002543280
- PAULE B: "Reappraisal of the concept of hormone therapy in metastatic prostate cancer and implications for treatment" EUROPEAN UROLOGY, vol. 47, no. 6, June 2005 (2005-06), pages 729-735, XP4941460
- SMITH P.C. ET AL: 'Anti-interleukin-6 monoclonal antibody induces regression of human prostate cancer xenografts in nude mice' THE PROSTATE vol. 48, 2001, pages 47 - 53, XP003012696
- ZAKI M.H. ET AL: 'CNTO 328, A monoclonal antibody to IL-6, inhibits human tumor-induced cachexia in nude mice' INTERNATIONAL JOURNAL OF CANCER vol. 111, 2004, pages 592 - 595, XP003012697
- SHIMAZAKI C. ET AL: 'Hito Kotsuzuishu Model to Ko hito IL-6 Juyotai Kotai no Ko Shuyo Koka' RINSHO KETSUEKI vol. 38, no. 4, 1997, pages 281 - 284, XP003012698
- BELLOMO R.: 'The cytokine network in the critically ill.' ANAESTHESIA AND INTENSIVE CARE vol. 30, no. 1, August 1992, pages 268 - 302
- TISDALE M.J.: 'Biology of Cachexia' JOURNAL OF THE NATIONAL CANCER INSTITUTE vol. 89, no. 23, 03 December 1997, pages 1763 - 1773
- CAMPO S. ET AL: 'Comparative activity of Sant7 and anti-IL-6, IL-6R monoclonal antibodies in a murine model of B-cell lymphoma.' CYTOKINE vol. 31, 2005, pages 368 - 374

## Description

### Technical Field

The present invention relates to agents for treating cachexia that accompanies prostate cancer, which comprise an IL-6 inhibitor as an active ingredient, and uses thereof.

### Background Art

Until now, prostate cancer has been treated with hormonal therapy, surgical therapy, radiation therapy, or chemotherapy, or combinations thereof. Hormonal therapy suppresses the production or activity of androgen which is involved in the growth of prostate cancer. The hormonal therapy is carried out by removing testicles that produce androgen, or by administering an LH-RH analog that acts on the pituitary gland and reduces the level of testosterone, an estrogen preparation, or an anti-androgen agent, etc. Hormonal therapy is the only therapeutic method available for treating advanced prostate cancer. However, many advanced prostate cancer patients acquire hormone resistance several years after starting hormonal therapy, and they struggle with the treatment. Thus, effective therapeutic methods for treating prostate cancer that has acquired resistance to the hormonal therapy are desired.

IL-6 is a cytokine called B-cell stimulating factor 2 (BSF2) or interferon β2. IL-6 was discovered as a differentiation factor involved in the activation of B-cell lymphocytes (Non-patent Document 1), and was later revealed to be a multifunctional cytokine that influences the function, of various cells (Non-patent Document 2). IL-6 has been reported to induce maturation of T lymphocyte cells (Non-patent Document 3).

IL-6 transmits its biological activity via two kinds of proteins on the cell. The first kind of protein is the IL-6 receptor, which is a ligand binding protein to which IL-6 binds; it has a molecular weight of about 80 kD (Non-patent Documents 4 and 5). The IL-6 receptor is present in a membrane-bound form that penetrates and is expressed on the cell membrane, and also as a soluble IL-6 receptor, which mainly consists of the extracellular region of the membrane-bound form.

The other kind of protein is the membrane protein gp130, which has a molecular weight of about 130 kD and is involved in non-ligand binding signal transduction. The biological activity of IL-6 is transmitted into the cell through formation of an IL-6/IL-6 receptor complex by IL-6 and Il-6 receptor followed by binding of the complex with gp130 (Non-patent Document 6).

IL-6 inhibitors are substances that inhibit the transmission of IL-6 biological activity. Currently, known IL-6 inhibitors include antibodies against IL-6 (anti-IL-6 antibodies), antibodies against IL-6 receptor (anti-IL-6 receptor antibodies), antibodies against gp130 (anti-gp130 antibodies), IL-6 variants, partial peptides of IL-6 or IL-6 receptor, and such.

There are several reports regarding anti-IL-6 receptor antibodies (Non-patent Documents 7 and 8, Patent Documents 1 to 3). One such report details a humanized PM-1 antibody, which is obtained by transplanting the complementarity determining region (CDR) of mouse antibody PM-1 (Non-patent Document 9), which is an anti-IL-6 receptor antibody, into a human antibody (Patent Document 4).

Recent reports show that IL-6 acts autocrinally or paracrinally to stimulate the growth of prostate cancer (Non-patent Document 10). The IL-6 overproduced by tumor is also suggested to be the major cause of cachexia in advanced prostate cancer patients.

Recent reports of *in vitro* experiments show that anti-IL-6 antibodies suppressed the growth of human prostate cancer cells (LNCaP, DU145, and PC3) under certain conditions (Non-patent Document 10). However, there is no report so far that suggests the growth of prostate cancer can be suppressed *in vivo* through specific inhibition of IL-6. It is well known that in diseases where cytokines such as IL-6 are involved, a particular cytokine forms a complicated network with other cytokines and multiple cytokines are known to have similar activities for the same cell type. Thus, it remains unclear as to whether IL-6 inhibitors are effective for treating prostate cancer.

Information on prior-art documents related to the present invention is described below:
[Non-patent Document 1] Hirano, T. et al., Nature (1986) 324, 73-76
[Non-patent Document 2] Akira, S. et al., Adv. in Immunology (1993) 54, 1-78
[Non-patent Document 3] Lotz, M. et al., J. Exp. Med. (1988) 167, 1253-1258
[Non-patent Document 4] Taga, T. et al., J. Exp. Med. (1987) 166, 967-981
[Non-patent Document 5] Yamasaki, K. et al., Science (1988) 241, 825-828
[Non-patent Document 6] Taga, T. et al., Cell (1989) 58, 573-581
[Non-patent Document 7] Novick, D. et al., Hybridoma (1991) 10, 137-146
[Non-patent Document 8] Huang, Y. W. et al., Hybridoma (1993) 12, 621-630
[Non-patent Document 9] Hirata, Y. et al., J. Immunol. (1989) 143, 2900-2906
[Non-patent Document 10] Okamoto et al., Cancer Res. 57(1), 141-6, 1997
[Patent Document 1] International Patent Application Publication No. WO 95/09873
[Patent Document 2] French Patent Application Publication No. FR 2694767
[Patent Document 3] U.S. Patent No. 5216128
[Patent Document 4] International Patent Application Publication No. WO 92/19759
Borsellino N et al: "Blocking signaling through the Gp130 receptor chain by interleukin-6 and oncostatin M inhibits PC-3 cell growth and sensitizes the tumor cells to etoposide and cisplatin-mediated cytotoxicity.", CANCER, vol. 85, no. 1, 1999, pages 134-144, describes the treatment of prostate cancer cell line PC-3 with Sant7, an IL-6 receptor binding inhibitor of IL-6 signalling.
In Tisdale MJ: "Biology of Cachexia" Journal of the National Cancer Institute, Vol 89, No 23, 1997, pages 1763-1773, it describes amongst others that in mice bearing colon-26 tumor an antibody to the IL-6 receptor reduced the loss of weight of the gastrocnemius muscle, but had no effect on the overall body weight or wasting of adipose tissue.

### Disclosure of the Invention

### [Problem to be Solved by the Invention]

The present invention was achieved in view of the above situation. An objective of the present invention is to provide prostate cancer therapeutic agents comprising an IL-6 inhibitor as an active ingredient.

### [Means for Solving the Problem]

In order to solve the above problem, the present inventors investigated the antitumor effects of anti-IL-6 receptor antibodies against prostate cancer.

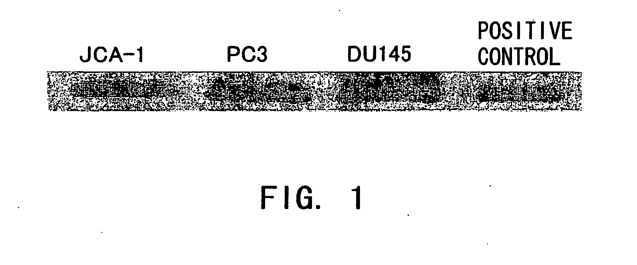

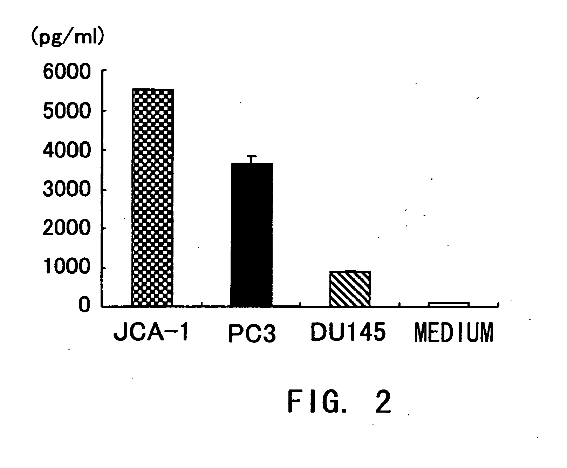

First, the present inventors examined by Western blotting whether IL-6 receptor was expressed in human prostate cancer cell lines PC3, DU145, and JCA-1. The result showed that IL-6 receptor was expressed in the above three cell lines (Fig. 1). In addition, IL-6 in the culture supernatants of the above-described cells were assayed by ELISA and the result showed that these cells produced IL-6 (Fig. 2).

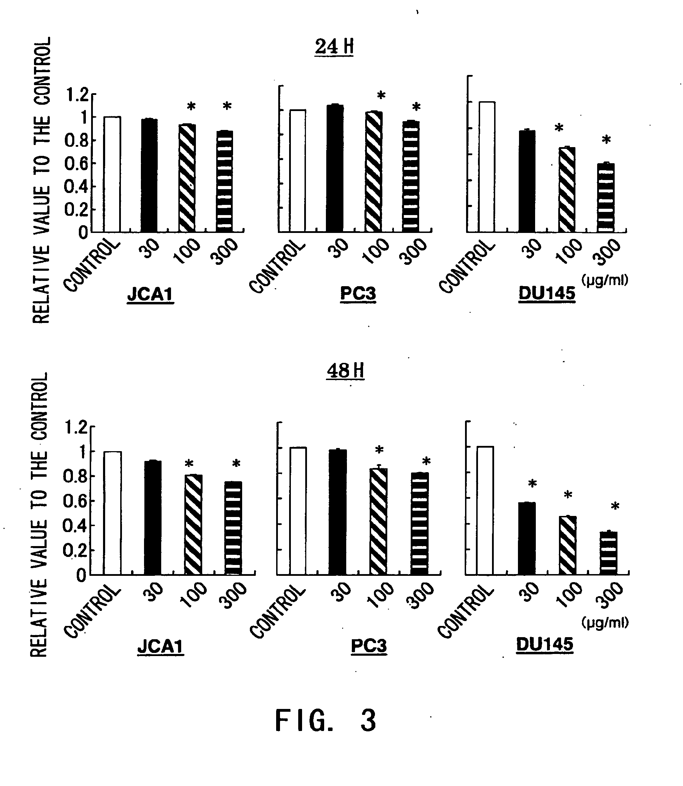

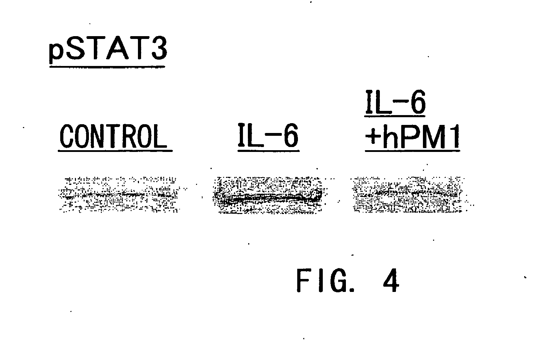

Next, the above-described cells were cultured *in vitro* in the presence of various concentrations of a humanized PM-1 antibody (hPM1). The antitumor effect was assessed after 24 and 48 hours of culture, and the result revealed that hPM1 shows cell growth suppression effect in a time- and concentration-dependent manner (Fig. 3). Whether hPM1 exerts the antitumor effect through the IL-6 receptor was examined by comparing the phosphorylation of STAT3 by Western blotting. The result showed that the phosphorylation of STAT3 in the prostate cancer cell lines was suppressed 60 minutes after hPM1 administration. It was also found that hPM1 suppressed the IL-6 stimulation-enhanced phosphorylation of STAT3 (Fig. 4).

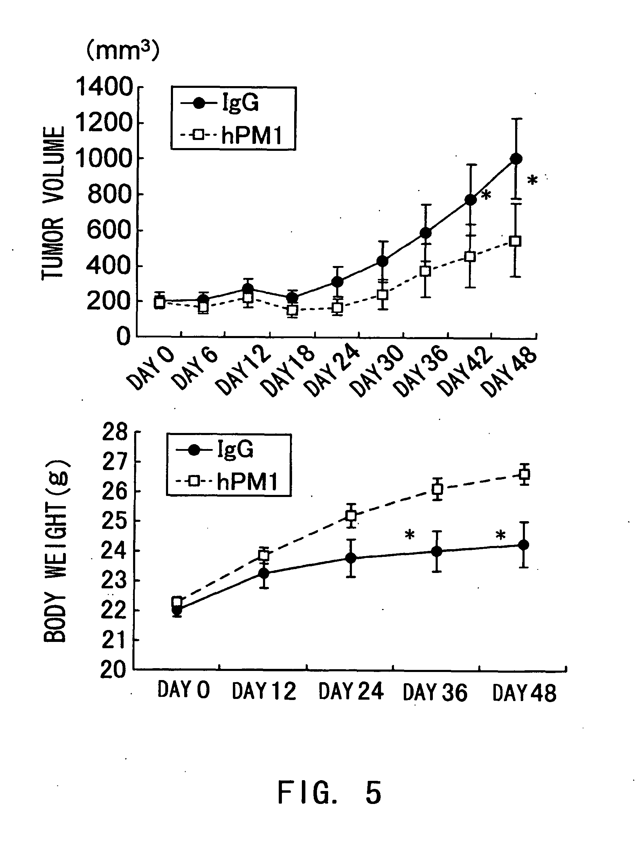

Furthermore, the humanized PM-1 antibody (hPM1) was administered to a cancer-bearing mouse model with subcutaneous tumor to confirm the *in vivo* antitumor effect of the anti-IL-6 receptor antibody. The result showed that the tumor growth and weight loss were significantly suppressed by administering hPM1 (Fig. 5).

The present inventors discovered for the first time that the growth of prostate cancer can be suppressed by administering an anti-IL-6 receptor antibody, and thus completed the present invention.

The present invention comprises the following embodiments:
1. A pharmaceutical composition comprising an antibody that recognizes an IL-6 receptor and blocks IL-6-mediated signal transduction as an active ingredient for use in treating cachexia that accompanies prostate cancer.
2. The pharmaceutical composition of embodiment 1 for use in treating cachexia that accompanies prostate cancer, wherein the antibody is a monoclonal antibody.
3. The pharmaceutical composition of embodiment 1 for use in treating cachexia that accompanies prostate cancer, wherein the antibody recognizes a human IL-6 receptor.
4. The pharmaceutical composition of embodiment 1 for use in treating cachexia that accompanies prostate cancer, wherein the antibody is a recombinant antibody.
5. The pharmaceutical composition of embodiment 4 for use in treating cachexia that accompanies prostate cancer, wherein the antibody is a chimeric, humanized, or human antibody.
6. The pharmaceutical composition of any one of embodiments 1 to 5, for the use according to embodiment 1, wherein the prostate cancer is hormone-refractory prostate cancer.
7. Use of an antibody that recognizes an IL-6 receptor and blocks IL-6-mediated signal transduction for the preparation of a pharmaceutical composition for treating cachexia that accompanies prostate cancer.
8. The use of embodiment 7, wherein the antibody is a monoclonal antibody.
9. The use of embodiment 7, wherein the antibody recognizes a human IL-6 receptor.
10. The use of embodiment 7, wherein the antibody is a recombinant antibody.
11. The use of embodiment 10, wherein the antibody is a chimeric, humanized, or human antibody.
12. The use of any one of embodiments 7 to 11, wherein the prostate cancer is hormone-refractory prostate cancer.

### Brief Description of the Drawings

Fig. 1 is a photograph of Western blot analysis showing that IL-6 receptor is expressed in human prostate cancer cell lines JCA-1, PC3, and DU145.
Fig. 2 is a graph showing that the IL-6 concentration in the culture supernatants of human prostate cancer cell lines JCA-1, PC3, and DU145 increased.
Fig. 3 is a graph showing that the humanized PM-1 antibody (hPM1) exhibits an *in vitro* antitumor effect against human prostate cancer cell lines JCA-1, PC3, and DU145. The horizontal axis values indicate the hPM1 concentration.
Fig. 4 is a photograph showing that the expression of pSTAT3 was enhanced by stimulating DU 145 cells with IL-6 for 60 minutes, and that the enhanced expression of pSTAT3 was suppressed by using hPM1.
Fig. 5 is a graph showing an *in vivo* antitumor effect of the humanized PM-1 antibody (hPM1) in the cancer-bearing mouse model with subcutaneous tumor, which was created by transplanting cells of the human prostate cancer cell line DU145 into SCID mice. The upper graph shows changes in the tumor size over time when the humanized PM-1 antibody or a control antibody (IgG) was administered to the cancer-bearing mice. The lower graph shows changes in the body weight over time when the humanized PM-1 antibody or a control antibody (IgG) was administered to the cancer-bearing mice.

### Best Mode for Carrying Out the Invention

The present inventors discovered that administration of an anti-IL-6 receptor antibody can suppress prostate cancer growth. The present invention is based on these findings.

The present invention relates to agents for treating cachexia that accompanies prostate cancer, which comprise an IL-6 inhibitor as an active ingredient.

Herein, an "IL-6 inhibitor" is a substance that blocks IL-6-mediated signal transduction and inhibits IL-6 biological activity. Preferably, the IL-6 inhibitor is a substance that has inhibitory function against the binding of IL-6, IL-6 receptor, or gp130.

The IL-6 inhibitors of the present invention are limited to anti-IL-6 receptor antibodies that block IL-6 mediated signal transduction.

The source of the antibodies is not particularly restricted in the present invention; however, the antibodies are preferably derived from mammals, and more preferably derived from humans.

Anti-IL-6 antibodies can be obtained as polyclonal or monoclonal antibodies using known means. In particular, monoclonal antibodies derived from mammals are preferred as the anti-IL-6 antibodies used in the present invention. Monoclonal antibodies derived from mammals include those produced from hybridomas and those produced by genetic engineering methods from hosts transformed with an expression vector that comprises an antibody gene. By binding to IL-6, the antibody inhibits IL-6 from binding to an IL-6 receptor and thus blocks the transmission of IL-6 biological activity into the cell.

Such antibodies include, MH166 (Matsuda, T. et al., Eur. J. Immunol. (1988) 18, 951-956), SK2 antibody (Sato, K. et al., transaction of the 21st Annual Meeting of the Japanese Society for Immunology (1991) 21, 166), and so on.

Basically, hybridomas that produce anti-IL-6 antibodies can be prepared using known techniques, as follows: Specifically, such hybridomas can be prepared by using IL-6 as a sensitizing antigen to carry out immunization using a conventional immunization method, fusing the obtained immune cells with known parent cells by a conventional cell fusion method, and screening for monoclonal antibody-producing cells using a conventional screening method.

More specifically, anti-IL-6 antibodies can be produced as follows: For example, human IL-6 for use as the sensitizing antigen for obtaining antibodies can be obtained using the IL-6 gene and/or amino acid sequences disclosed in Eur. J. Biochem. (1987) 168, 543-550; J. Immunol. (1988) 140, 1534-1541; and/orAgr. Biol. Chem. (1990) 54,2685-2688.

After transforming an appropriate host cell with a known expression vector system inserted with an IL-6 gene sequence, the desired IL-6 protein is purified using known methods from the inside of the host cell or from the culture supernatant. This purified IL-6 protein may be used as a sensitizing antigen. Alternatively, a fusion protein of the IL-6 protein and another protein may be used as a sensitizing antigen.

Anti-IL6 receptor antibodies used for the present invention can be obtained as polyclonal or monoclonal antibodies by using known methods. In particular, the anti-IL-6 receptor antibodies used in the present invention are preferably monoclonal antibodies derived from mammals. The monoclonal antibodies derived from mammals include those produced from hybridomas and those produced using genetic engineering methods from hosts transformed with an expression vector that comprises an antibody gene. By binding to an IL-6 receptor, the antibody inhibits IL-6 from binding to the IL-6 receptor, and thus blocks the transmission of IL-6 biological activity into the cell.

Such antibodies include, MR16-1 antibody (Tamura, T. et al., Proc. Natl. Acad. Sci. USA (1993) 90, 11924-11928); PM-1 antibody (Hirata, Y et al., J. Immunol. (1989) 143, 2900-2906); AUK12-20 antibody, AUK64-7 antibody and AUK146-15 antibody (WO 92/19759); and so on. Of these, the PM-1 antibody can be exemplified as a preferred monoclonal antibody against the human IL-6 receptor, and the MR16-1 antibody as a preferred monoclonal antibody against the mouse IL-6 receptor.

Basically, hybridomas producing an anti-IL-6 receptor monoclonal antibody can be prepared using known techniques, as follows: Specifically, such hybridomas can be prepared by using an IL-6 receptor as the sensitizing antigen to carry out immunization by a conventional immunization method, fusing the obtained immune cells with a known parent cell using a conventional cell fusion method, and screening for monoclonal antibody-producing cells using a conventional screening method.

More specifically, anti-IL-6 receptor antibodies can be produced as follows: For example, a human IL-6 receptor or mouse IL-6 receptor for use as a sensitizing antigen for obtaining antibodies can be obtained by using the IL-6 receptor genes and/or amino acid sequences disclosed in European Patent Application Publication No. EP 325474 and Japanese Patent Application Kokai Publication No. (JP-A) H03-155795, respectively.

There are two kinds of IL-6 receptor proteins: one expressed on the cell membrane and the other separated from the cell membrane (soluble IL-6 receptors) (Yasukawa, K. et al., J. Biochem. (1990) 108, 673-676). The soluble IL-6 receptor essentially consists of the extracellular region of the cell membrane-bound IL-6 receptor, and differs from the membrane-bound IL-6 receptor in that it lacks the transmembrane region or both the transmembrane and intracellular regions. Any IL-6 receptor may be employed as an IL-6 receptor protein, so long as it can be used as a sensitizing antigen for producing an anti-IL-6 receptor antibody used in the present invention.

After transforming an appropriate host cell with a known expression vector system inserted with an IL-6 receptor gene sequence, the desired IL-6 receptor protein is purified from the inside of the host cell or from the culture supernatant using a known method. This purified IL-6 receptor protein may be used as a sensitizing antigen. Alternatively, a cell expressing the IL-6 receptor or a fusion protein of the IL-6 receptor protein and another protein may be used as a sensitizing antigen.

Anti-gp130 antibodies can be obtained as polyclonal or monoclonal antibodies by using known methods. In particular, the anti-gp130 antibodies used in the present disclosure are preferably monoclonal antibodies derived from mammals. Mammal-derived monoclonal antibodies include those produced from hybridomas and those produced using genetic engineering methods from hosts transformed with an expression vector that comprises an antibody gene. By binding to gp130, the antibody inhibits gp130 from binding to the IL-6/IL-6 receptor complex, and thus blocks transmission of IL-6 biological activity into the cell.

Such antibodies include, AM64 antibody (JP-A (Kokai) H03-219894); 4B11 antibody and 2H4 antibody (US 5571513); B-S12 antibody and B-P8 antibody (JP-A (Kokai) H08-291199); and so on.

Basically, anti-gp130 monoclonal antibody-producing hybridomas can be prepared using known techniques, as follows: Specifically, such hybridomas can be prepared by using gp130 as a sensitizing antigen to carry out the immunization using a conventional immunization method, fusing the obtained immune cells with a known parent cell by a conventional cell fusion method, and screening for monoclonal antibody-producing cells using a conventional screening method.

More specifically, monoclonal antibodies can be produced as follows: For example, gp130 for use as a sensitizing antigen for obtaining antibodies can be obtained using the gp130 gene and/or amino acid sequence disclosed in European Patent Application Publication No. EP 411946.

After transforming an appropriate host cell with a known expression vector system inserted with a gp130 gene sequence, the desired gp130 protein is purified by a known method from the inside of the host cell or from the culture supernatant. This purified gp130 protein may be used as a sensitizing antigen. Alternatively, a cell expressing gp130 or a fusion protein of the gp130 protein and another protein may be used as a sensitizing antigen.

Mammals to be immunized with a sensitizing antigen are not particularly limited, but are preferably selected in consideration of compatibility with the parent cell used for cell fusion. Generally, rodents such as mice, rats, and hamsters are used.

Animals are immunized with sensitizing antigens according to known methods. For example, as a general method, animals are immunized by intraperitoneal or subcutaneous injection of a sensitizing antigen. Specifically, the sensitizing antigen is preferably diluted or suspended in an appropriate amount of phosphate-buffered saline (PBS), physiological saline or such, mixed with an appropriate amount of a general adjuvant (*e.g*., Freund's complete adjuvant), emulsified, and then administered to a mammal several times, every four to 21 days. In addition, an appropriate carrier may be used for immunization with a sensitizing antigen.

Following such immunization, an increased level of a desired antibody in serum is confirmed and then immune cells are obtained from the mammal for cell fusion. Preferred immune cells for cell fusion include, in particular, spleen cells.

The mammalian myeloma cells used as parent cells, *i.e*. as partner cells to be fused with the above immune cells, include various known cell strains, for example, P3X63Ag8.653 (Kearney, J. F. et al., J. Immunol (1979) 123, 1548-1550), P3X63Ag8U.1 (Current Topics in Microbiology and Immunology (1978) 81, 1-7), NS-1 (Kohler, G. and Milstein, C., Eur. J. Immunol. (1976) 6, 511-519), MPC-11 (Margulies, D. H. et al., Cell (1976) 8, 405-415), SP2/0 (Shulman, M. et al., Nature (1978) 276, 269-270), F0 (de St. Groth, S. F. et al., J. Immunol. Methods (1980) 35, 1-21); S194 (Trowbridge, I. S., J. Exp. Med. (1978) 148, 313-323), R210 (Galfre, G. et al., Nature (1979) 277, 131-133), and such.

Basically, cell fusion of the aforementioned immune cells and myeloma cells can be performed using known methods, for example, the method of Milstein *et al.* (Kohler, G. and Milstein, C., Methods Enzymol. (1981) 73, 3-46), and such.

More specifically, the aforementioned cell fusion is achieved in general nutrient culture medium in the presence of a cell fusion enhancing agent. For example, polyethylene glycol (PEG), Sendai virus (HVJ), and such are used as fusion enhancing agents. Further, to enhance fusion efficiency, auxiliary agents such as dimethyl sulfoxide may be added depending on needs.

The ratio of immune cells to myeloma cells used is preferably, for example, 1 to 10 immune cells for each myeloma cell. The culture medium used for the aforementioned cell fusion is, for example, the RPMI1640 or MEM culture medium, which are suitable for proliferation of the aforementioned myeloma cells. A general culture medium used for culturing this type of cell can also be used. Furthermore, serum supplements such as fetal calf serum (FCS) can be used in combination.

For cell fusion, the fusion cells (hybridomas) of interest are formed by mixing predetermined amounts of an aforementioned immune cell and myeloma cell in an aforementioned culture medium, and then adding and mixing a concentration of 30% to 60% (w/v) PEG solution (*e.g*., a PEG solution with a mean molecular weight of about 1,000 to 6,000) pre-heated to about 37°C. Then, cell fusion agents and such that are unsuitable for the growth of hybridomas can be removed by repeatedly adding an appropriate culture medium and then removing the supernatant by centrifugation.

The above hybridomas are selected by culturing cells in a general selection culture medium, for example, HAT culture medium (a culture medium containing hypoxanthine, aminopterin, and thymidine). Culture in HAT culture medium is continued for a sufficient period, generally several days to several weeks, to kill cells other than the hybridomas of interest (unfused cells). Then, a standard limited dilution method is performed to screen and clone hybridomas that produce an antibody of interest.

In addition to the methods for immunizing non-human animals with antigens for obtaining the aforementioned hybridomas, desired human antibodies with the activity of binding to a desired antigen or antigen-expressing cell can be obtained by sensitizing a human lymphocyte with a desired antigen protein or antigen-expressing cell *in vitro,* and fusing the sensitized B lymphocyte with a human myeloma cell (*e.g.,* U266) (see, Japanese Patent Application Kokoku Publication No. (JP-B) H01-59878 (examined, approved Japanese patent application published for opposition)). Further, a desired human antibody can be obtained by administering an antigen or antigen-expressing cell to a transgenic animal that has a repertoire of human antibody genes, and then following the aforementioned method (see, International Patent Application Publication Nos. WO 93/12227, WO 92/03918, WO 94/02602, WO 94/25585, WO 96/34096, and WO 96/33735).

The thus-prepared hybridomas which produce monoclonal antibodies can be subcultured in a conventional culture medium and stored in liquid nitrogen for a long period.

When obtaining monoclonal antibodies from the aforementioned hybridomas, the following methods may be employed: (1) methods where the hybridomas are cultured according to conventional methods and the antibodies are obtained as a culture supernatant; (2) methods where the hybridomas are proliferated by administering them to a compatible mammal and the antibodies are obtained as ascites; and so on. The former method is preferred for obtaining antibodies with high purity, and the latter is preferred for large-scale antibody production.

For example, anti-IL-6 receptor antibody-producing hybridomas can be prepared by the method disclosed in JP-A (Kokai) H03-139293. Such hybridomas can be prepared by injecting a PM-1 antibody-producing hybridoma into the abdominal cavity of a BALB/c mouse, obtaining ascites, and then purifying a PM-1 antibody from the ascites; or by culturing the hybridoma in an appropriate medium (*e.g*., RPMI1640 medium containing 10% fetal bovine serum, and 5% BM-Condimed H1 (Boehringer Mannheim); hybridoma SFM medium (GIBCO-BRL); PFHM-II medium (GIBCO-BRL), *etc.*) and then obtaining PM-1 antibody from the culture supernatant.

Recombinant antibodies can be used as the monoclonal antibodies of the present invention, wherein the antibodies are produced using genetic recombination techniques by cloning an antibody gene from a hybridoma, inserting the gene into an appropriate vector, and then introducing the vector into a host (see, for example, Borrebaeck, C. A. K. and Larrick, J. W., Therapeutic Monoclonal Antibodies, published in the United Kingdom by Macmillan Publishers Ltd, 1990).

More specifically, mRNAs coding for antibody variable (V) regions are isolated from cells that produce antibodies of interest, such as hybridomas. mRNAs can be isolated by preparing total RNAs according to known methods, such as the guanidine ultracentrifugation method (Chirgwin, J. M. et al., Biochemistry (1979) 18, 5294-5299) and the AGPC method (Chomczynski, P. et al., Anal. Biochem. (1987) 162, 156-159), and preparing mRNAs using an mRNA Purification Kit (Pharmacia) and such. Alternatively, mRNAs can be directly prepared using a QuickPrep mRNA Purification Kit (Pharmacia).

cDNAs of the antibody V regions are synthesized from the obtained mRNAs using reverse transcriptase. cDNAs may be synthesized using an AMV Reverse Transcriptase First-strand cDNA Synthesis Kit and so on. Further, to synthesize and amplify the cDNAs, the 5'-RACE method (Frohman, M. A. et al., Proc. Natl. Acad. Sci. USA (1988) 85, 8998-9002; Belyavsky, A. et al., Nucleic Acids Res. (1989) 17, 2919-2932) using 5'-Ampli FINDER RACE Kit (Clontech) and PCR may be employed. A DNA fragment of interest is purified from the obtained PCR products and then ligated with a vector DNA. Then, a recombinant vector is prepared using the above DNA and introduced into *Escherichia coli* or such, and then its colonies are selected to prepare a desired recombinant vector. The nucleotide sequence of the DNA of interest is confirmed by, for example, the deoxy method.

When a DNA encoding the V region of an antibody of interest is obtained, the DNA is ligated with a DNA that encodes a desired antibody constant region (C region), and inserted into an expression vector. Alternatively, a DNA encoding an antibody V region may be inserted into an expression vector comprising a DNA of an antibody C region.

To produce an antibody to be used in the present invention, as described below, an antibody gene is inserted into an expression vector such that it is expressed under the control of an expression regulating region, for example, an enhancer and promoter. Then, the antibody can be expressed by transforming a host cell with this expression vector.

In the present invention, to reduce heteroantigenicity against humans and such, artificially modified genetic recombinant antibodies, for example, chimeric antibodies, humanized antibodies, or human antibodies, can be used. These modified antibodies can be prepared using known methods.

A chimeric antibody can be obtained by ligating a DNA encoding an antibody V region, obtained as above, with a DNA encoding a human antibody C region, then inserting the DNA into an expression vector and introducing it into a host for production (see, European Patent Application Publication No. EP 125023; International Patent Application Publication No. WO 92/19759). This known method can be used to obtain chimeric antibodies useful for the present invention.

Humanized antibodies are also referred to as reshaped human antibodies, and are antibodies wherein the complementarity determining regions (CDRs) of an antibody from a mammal other than human (*e.g*., a mouse antibody) are transferred into the CDRs of human antibodies. General methods for this gene recombination are also known (see, European Patent Application Publication No. EP 125023, International Patent Application Publication No. WO 92/19759).

More specifically, DNA sequences designed such that the CDRs of a mouse antibody are ligated with the framework regions (FRs) of a human antibody are synthesized by PCR from several oligonucleotides produced to contain overlapping portions at their termini. The obtained DNA is ligated with a human antibody C region-encoding DNA and then inserted into an expression vector. The expression vector is introduced into a host to produce the humanized antibody (see, European Patent Application Publication No. EP 239400, International Patent Application Publication No WO 92/19759).

The human antibody FRs to be ligated via the CDRs are selected so that the CDRs form suitable antigen binding sites. The amino acid(s) within the FRs of the antibody variable regions may be substituted as necessary so that the CDRs of the reshaped human antibody form an appropriate antigen binding site (Sato, K. et al., Cancer Res. (1993) 53, 851-856).

Human antibody C regions are used for the chimeric and humanized antibodies, and include Cγ. For example, Cγ1, Cγ2, Cγ3, or Cγ4 may be used. Furthermore, to improve the stability of the antibodies or their production, the human antibody C regions may be modified.

Chimeric antibodies consist of the variable region of an antibody derived from a non-human mammal and the constant region of an antibody derived from a human; humanized antibodies consist of the CDRs of an antibody derived from a non-human mammal and the framework regions and constant regions derived from a human antibody. Both have reduced antigenicity in the human body, and are thus useful as antibodies for use in the present invention.

Preferred specific examples of humanized antibodies for use in the present invention include the humanized PM-1 antibody (see, International Patent Application Publication No. WO 92/19759).

Furthermore, in addition to the aforementioned methods for obtaining human antibodies, techniques for obtaining human antibodies by panning using a human antibody library are also known. For example, the variable regions of human antibodies can be expressed on phage surfaces as single chain antibodies (scFv) by using the phage display method, and antigen-binding phages can then be selected. By analyzing the genes of the selected phages, DNA sequences coding for the human antibody variable regions that bind to the antigen can be determined. Once the DNA sequence of an scFv that binds to the antigen is revealed, an appropriate expression vector comprising the sequence can be constructed to obtain an human antibody. These methods are already known, and the publications of WO 92/01047, WO 92/20791, WO93/06213, WO 93/11236, WO 93/19172, WO 95/01438, and WO 95/15388 can be used as reference.

The antibody genes constructed above can be expressed according to conventional methods. When a mammalian cell is used, the antibody gene can be expressed using a DNA in which the antibody gene to be expressed is functionally ligated to a useful commonly used promoter and a poly A signal downstream of the antibody gene, or a vector comprising the DNA. Examples of a promoter/enhancer include the human cytomegalovirus immediate early promoter/enhancer.

Furthermore, other promoters/enhancers that can be used for expressing the antibodies for use in the present invention include viral promoters/enhancers from retroviruses, polyoma viruses, adenoviruses, simian virus 40 (SV40), and such; and also include mammalian cell-derived promoters/enhancers such as human elongation factor 1α (HEF1α).

For example, when the SV40 promoter/enhancer is used, the expression can be easily performed by following the method by Mulligan *et al.* (Mulligan, R. C. et al., Nature (1979) 277, 108-114). Alternatively, in the case of the HEF1α promoter/enhancer, the method by Mizushima *et al.* (Mizushima, S. and Nagata S., Nucleic Acids Res. (1990) 18, 5322) can be used.

When *E*. *coli* is used, an antibody gene can be expressed by functionally ligating a conventional promoter, a signal sequence for antibody secretion, and the antibody gene to be expressed. Examples of the promoter include a lacZ promoter, araB promoter and such. When a lacZ promoter is used, genes can be expressed according to the method of Ward *et al.* (Ward, E. S. et al., Nature (1989) 341, 544-546; Ward, E. S. et al., FASEB J. (1992) 6, 2422-2427); and the araB promoter may be used according to the method of Better *et al.* (Better, M. et al., Science (1988) 240, 1041-1043).

When the antibody is produced into the periplasm of *E*. *coli,* the pel B signal sequence (Lei, S. P. et al., J. Bacteriol. (1987) 169,4379-4383) may be used as a signal sequence for antibody secretion. The antibodies produced into the periplasm are isolated, and then used after appropriately refolding the antibody structure (see, for example, WO 96/30394).

As the replication origin, those derived from SV40, polyoma virus, adenovirus, bovine papilloma virus (BPV) and such may be used. In addition, to enhance the gene copy number in a host cell system, the expression vector may comprise the aminoglycoside phosphotransferase (APH) gene, thymidine kinase (TK) gene, *E*. *coli* xanthine-guanine phosphoribosyltransferase (Ecogpt) gene, dihydrofolate reductase (dhfr) gene, or such as a selection marker.

Any production system may be used to prepare the antibodies for use in the present invention. The production systems for antibody preparation include *in vitro* and *in vivo* production systems. *In vitro* production systems include those using eukaryotic cells or prokaryotic cells.

Production systems using eukaryotic cells include those using animal cells, plant cells, or fungal cells. Such animal cells include (1) Mammalian cells, for example, CHO, COS, myeloma, baby hamster kidney (BHK), HeLa, Vero, and such; (2) amphibian cells, for example, *Xenopus* oocyte; and (3) insect cells, for example, sf9, sf21, Tn5, and such. Known plant cells include cells derived from *Nicotiana tabacum,* which may be cultured as a callus. Known fungal cells include yeasts such as *Saccharomyces* (*e.g., S. cerevisiae*), mold fungi such as *Aspergillus* (*e.g*., *A. niger*)*,* and such.

Production systems using prokaryotic cells include those using bacterial cells. Known bacterial cells include *E. coli* and *Bacillus subtilis.*

Antibodies can be obtained by using transformation to introduce an antibody gene of interest into these cells, and then culturing the transformed cells *in vitro.* Cultures are conducted according to known methods. For example, DMEM, MEM, RPMI1640, IMDM may be used as the culture medium, and serum supplements such as FCS may be used in combination. Further, cells introduced with antibody genes may be transferred into the abdominal cavity or such of an animal to produce the antibodies *in vivo.*

On the other hand, *in vivo* production systems include those using animals or plants. Production systems using animals include those that use mammals or insects.

Mammals that can be used include goats, pigs, sheep, mice, bovines and such (Vicki Glaser, SPECTRUM Biotechnology Applications, 1993). Further, insects that can be used include silkworms. When using plants, tobacco may be used, for example.

An antibody gene is introduced into these animals or plants, the antibody is produced in the body of the animals or plants, and this antibody is then recovered. For example, an antibody gene can be prepared as a fusion gene by inserting it into the middle of a gene encoding a protein such as goat β casein, which is uniquely produced into milk. DNA fragments comprising the fusion gene, which includes the antibody gene, are injected into goat embryos, and the embryos are introduced into female goats. The desired antibody is obtained from milk produced by the transgenic animals born to the goats that received the embryos, or produced from progenies of these animals. The transgenic goats can be given hormones to increase the volume of milk containing the desired antibody that they produce (Ebert, K. M. et al., Bio/Technology (1994) 12, 699-702).

When silkworms are used, the silkworms are infected with a baculovirus inserted with a desired antibody gene, and the desired antibody is obtained from the body fluids of these silkworms (Maeda, S. et al., Nature (1985) 315, 592-594). Moreover, when tobacco is used, the desired antibody gene is inserted into a plant expression vector (*e.g*., pMON530) and the vector is introduced into bacteria such as *Agrobacterium tumefaciens.-* This bacterium is used to infect tobacco (*e.g., Nicotiana tabacum*) such that desired antibodies can be obtained from the leaves of this tobacco (Julian, K. -C. Ma et al., Eur. J. Immunol. (1994) 24, 131-138).

When producing antibodies using *in vitro* or *in vivo* production systems, as described above, DNAs encoding an antibody heavy chain (H chain) and light chain (L chain) may be inserted into separate expression vectors and a host is then co-transformed with the vectors. Alternatively, the DNAs may be inserted into a single expression vector for transforming a host (see International Patent Application Publication No. WO 94/11523).

The antibodies used in the present invention may be antibody fragments or modified products thereof, so long as they can be suitably used in the present invention. For example, antibody fragments include Fab, F(ab')2, Fv, and single chain Fv (scFv), in which the Fvs of the H and L chains are linked via an appropriate linker.

Specifically, the antibody fragments are produced by treating antibodies with enzymes, for example, papain or pepsin, or alternatively, genes encoding these fragments are constructed, introduced into expression vectors, and these are expressed in appropriate host cells (see, for example, Co, M. S. et al., J. Immunol. (1994) 152, 2968-2976; Better, M. & Horwitz, A. H., Methods in Enzymology (1989) 178, 497-515; Plueckthun, A. & Skerra, A., Methods in Enzymology (1989) 178, 497-515; Lamoyi, E., Methods in Enzymology (1989) 121, 652-663; Rousseaux, J. et al., Methods in Enzymology (1989) 121, 663-666; Bird, R. E. et al., TIBTECH (1991) 9, 132-137).

An scFv can be obtained by linking the H-chain V region and the L-chain V region of an antibody. In the scFv, the H-chain V region and the L-chain V region are linked via a linker, - preferably via a peptide linker (Huston, J. S. et al., Proc. Natl. Acad. Sci. USA (1988) 85, 5879-5883). The V regions of the H and L chains in an scFv may be derived from any of the antibodies described above. Peptide linkers for linking the V regions include, for example, arbitrary single chain peptides consisting of 12 to 19 amino acid residues.

An scFv-encoding DNA can be obtained by using a DNA encoding an H chain or a V region and a DNA encoding an L chain or a V region of the aforementioned antibodies as templates, using PCR to amplify a DNA portion that encodes the desired amino acid sequence in the template sequence and uses primers that define the termini of the portion, and then further amplifying the amplified DNA portion with a DNA that encodes a peptide linker portion and primer pairs that link both ends of the linker to the H chain and L chain.

Once an scFv-encoding DNA has been obtained, an expression vector comprising the DNA and a host transformed with the vector can be obtained according to conventional methods. In addition, scFv can be obtained according to conventional methods using the host.

As above, these antibody fragments can be produced from the host by obtaining and expressing their genes. Herein, an "antibody" encompasses such antibody fragments.

Antibodies bound to various molecules, such as polyethylene glycol (PEG), may also be used as modified antibodies. Herein, an "antibody" encompasses such modified antibodies. These modified antibodies can be obtained by chemically modifying the obtained antibodies. Such methods are already established in the art.

Antibodies produced and expressed as above can be isolated from the inside or outside of the cells or from the hosts, and then purified to homogeneity. The antibodies for use in the present invention can be isolated and/or purified using affinity chromatography. Columns to be used for the affinity chromatography include, for example, protein A columns and protein G columns. Carriers used for the protein A columns include, for example, HyperD, POROS, Sepharose FF and such. In addition to the above, other methods used for the isolation and/or purification of common proteins may be used, and are not limited in any way.

For example, the antibodies used for the present invention may be isolated and/or purified by appropriately selecting and combining chromatographies in addition to affinity chromatography, filters, ultrafiltration, salting-out, dialysis, and such. Chromatographies include, for example, ion-exchange chromatography, hydrophobic chromatography, gel filtration, and such. These chromatographies can be applied to high performance liquid chromatography (HPLC). Alternatively, reverse phase HPLC may be used.

The concentration of the antibodies obtained as above can be determined by absorbance measurement, ELISA, or such. Specifically, absorbance is determined by appropriately diluting the antibody solution with PBS(-), measuring absorbance at 280 nm, and calculating the concentration (1.35 OD = 1 mg/ml). Alternatively, when using ELISA, the measurement can be performed as follows: Specifically, 100 µl of goat anti-human IgG (TAG) diluted to 1 µg/ml with 0.1 M bicarbonate buffer (pH 9.6) is added to a 96-well plate (Nunc) and incubated overnight at 4°C to immobilize the antibody. After blocking, 100 µl of an appropriately diluted antibody of the present invention or an appropriately diluted sample comprising the antibody, and human IgG (CAPPEL) are added as a standard, and incubated for one hour at room temperature.

After washing, 100 µl of 5,000x diluted alkaline phosphatase-labeled anti-human IgG (BIO SOURCE) is added and incubated for one hour at room temperature. After another wash, substrate solution is added and incubated, and the absorbance at 405 nm is measured using a Microplate Reader Model 3550 (Bio-Rad) to calculate the concentration of the antibody of interest.

The IL-6 variants are substances with the activity of binding to an IL-6 receptor and which do not transmit IL-6 biological activity. That is, the IL-6 variants compete with IL-6 to bind to IL-6 receptors, but fail to transmit IL-6 biological activity, and hence the block IL-6-mediated signal transduction.

The IL-6 variants are produced by introducing mutation(s) by substituting amino acid residues in the amino acid sequence of IL-6. The origin of IL-6 used as the base of the IL-6 variants is not limited, but is preferably human IL-6 in consideration of antigenicity and such.

More specifically, amino acid substitutions are performed by predicting the secondary structure of the IL-6 amino acid sequence using known molecular modeling programs (*e.g*., WHATIF; Vriend et al., J. Mol. Graphics (1990) 8, 52-56), and further assessing the influence of the substituted amino acid residue(s) on the whole molecule. After determining the appropriate amino acid residue to be substituted, commonly performed PCR methods are carried out using a nucleotide sequence encoding a human IL-6 gene as a template, and mutations are introduced to cause amino acids substitutions, and thus genes encoding IL-6 variants are obtained. If needed, this gene is inserted into an appropriate expression vector, and the IL-6 variant can be obtained by applying the aforementioned methods for expression, production, and purification of recombinant antibodies.

Specific examples of the IL-6 variants are disclosed in Brakenhoff et al., J. Biol. Chem. (1994) 269, 86-93, Savino et al., EMBO J. (1994) 13, 1357-1367, WO 96/18648, and WO 96/17869.

The partial peptides of IL-6 and of the IL-6 receptor are substances with the activity of binding to the IL-6 receptor and to IL-6, respectively, and which do not transmit IL-6 biological activity. Namely, by binding to and capturing an IL-6 receptor or IL-6, the IL-6 partial peptides or IL-6 receptor partial peptides can specifically inhibit IL-6 from binding to the IL-6 receptor. As a result, the biological activity of IL-6 is not transmitted, and IL-6-mediated signal transduction is blocked.

The partial peptides of IL-6 or IL-6 receptor are peptides that comprise part or all of the amino acid sequence of the region of the IL-6 or IL-6 receptor amino acid sequence that is involved in the binding between the IL-6 and IL-6 receptor. Such peptides usually comprise ten to 80, preferably 20 to 50, more preferably 20 to 40 amino acid residues.

The IL-6 partial peptides or IL-6 receptor partial peptides can be produced according to generally known methods, for example, genetic engineering techniques or peptide synthesis methods, by specifying the region of the IL-6 or IL-6 receptor amino acid sequence that is involved in the binding between the IL-6 and IL-6 receptor, and using a portion or entirety of the amino acid sequence of the specified region.

When preparing an IL-6 partial peptide or IL-6 receptor partial peptide using genetic engineering methods, a DNA sequence encoding the desired peptide is inserted into an expression vector, and then the peptide can be obtained by applying the aforementioned methods for expressing, producing, and purifying recombinant antibodies.

When producing an IL-6 partial peptide or IL-6 receptor partial peptide by using peptide synthesis methods, generally used peptide synthesis methods, for example, solid phase synthesis methods or liquid phase synthesis methods, may be used.

Specifically, the peptides can be synthesized according to the method described in "Continuation of Development of Pharmaceuticals, Vol. 14, Peptide Synthesis (in Japanese) (ed. Haruaki Yajima, 1991, Hirokawa Shoten)". As a solid phase synthesis method, for example, the following method can be employed: the amino acid corresponding to the C terminus of the peptide to be synthesized is bound to a support that is insoluble in organic solvents, then the peptide strand is elongated by alternately repeating (1) the reaction of condensing amino acids, whose α-amino groups and branch chain functional groups are protected with appropriate protecting groups, one at a time in a C- to N-terminal direction; and (2) the reaction of removing the protecting groups from the α-amino groups of the resin-bound amino acids or peptides. Solid phase peptide synthesis is broadly classified into the Boc method and the Fmoc method, depending on the type of protecting groups used.

After synthesizing a protein of interest as above, deprotection reactions are carried out, then the peptide strand is cleaved from its support. For the cleavage reaction of the peptide strand, hydrogen fluoride or trifluoromethane sulfonic acid is generally used for the Boc method, and TFA is generally used for the Fmoc method. In the Boc method, for example, the above-mentioned protected peptide resin is treated with hydrogen fluoride in the presence of anisole. Then, the peptide is recovered by removing the protecting groups and cleaving the peptide from its support. By freeze-drying the recovered peptide, a crude peptide can be obtained. In the Fmoc method, on the other hand, the deprotection reaction and the reaction to cleave the peptide strand from the support can be performed in TFA using a method similar to those described above, for example.

Obtained crude peptides can be separated and/or purified by applying HPLC. Elution may be performed under optimum conditions using a water-acetonitrile solvent system, which is generally used for protein purification. The fractions corresponding to the peaks of the obtained chromatographic profile are collected and freeze-dried. Thus, purified peptide fractions are identified by molecular weight analysis via mass spectrum analysis, amino acid composition analysis, amino acid sequence analysis, or such.

Specific examples of IL-6 partial peptides and IL-6 receptor partial peptides are disclosed in JP-A (Kokai) H02-188600, JP-A (Kokai) H07-324097, JP-A (Kokai) H08-311098, and United States Patent Publication No. US 5210075.

The antibodies used in the present invention may also be conjugated antibodies that are bound to various molecules, such as polyethylene glycol (PEG), radioactive substances, and toxins. Such conjugated antibodies can be obtained by chemically modifying the obtained antibodies. Methods for modifying antibodies are already established in the art. The "antibodies" of the present invention encompass these conjugated antibodies.

The prostate cancer therapeutic agents can be used to treat prostate cancer. Herein, prostate cancer refers to canceration of prostatic glandular cells.

Herein, "treatment of prostate cancer" refers to suppression of prostate cancer growth, suppression or prevention of prostate cancer metastasis, or suppression of cachexia or such that accompanies prostate cancer progression. The above treatment also includes suppression of post-surgery prostate cancer recurrence.

Suppression of prostate cancer growth can be confirmed by examining the shape and condition of prostate by rectal examination, assessing the primary lesion and metastatic lesion of prostate cancer by diagnostic imaging, or measuring the level of prostate specific antigen (PSA) (PSA test). It is known that the level of PSA is elevated as prostate cancer progresses. In other words, if the PSA level is decreased by administering the agents of the present disclosure, the growth of prostate cancer can be considered to be suppressed.

Alternatively, whether prostate cancer growth is suppressed can also be confirmed by assessing the size (or volume) of a primary lesion and metastatic lesion of prostate cancer by diagnostic imaging or such. The prostate cancer growth is considered to be suppressed when the prostate cancer volume is reduced by administering an agent of the present disclosure. The prostate cancer volume can be measured by known methods as well as methods described in the Examples.

Alternatively, whether prostate cancer growth is suppressed can also be confirmed by histopathological examination (biopsy) of tissue samples collected from the prostate. Classification based on the Gleason score is most frequently used to indicate the grade of prostate cancer malignancy. Tissues collected by biopsy are examined histopathologically and scores 2 to 10 were used to classify the cancer. A greater score means higher malignancy grade, and thus higher metastatic probability. Typically, if the cancer has a low malignancy grade and a Gleason score of 5 or less within a narrow area in the prostate, the prognosis is thought to be favorable. This tendency does not depend on the patient's age. By contrast, the prognosis of cancer with a Gleason score of 7 or more is thought to be poor. In other words, the prostate cancer growth is considered to be suppressed when the Gleason score is decreased by administering the agents of the present invention.

As prostate cancer progresses, cachexia such as weight loss and anemia develops and the overall condition deteriorates. When the overall condition is improved by administering an agent of the present invention, the agent is considered useful for prostate cancer patients.

The activity of IL-6 inhibitors in inhibiting the transduction of IL-6 signals can be evaluated by conventional methods. Specifically, IL-6 is added to cultures of IL-6-dependent human myeloma cell lines (S6B45 and KPMM2), human Lennert T lymphoma cell line KT3, or IL-6-dependent cell line MH60.BSF2; and the ³H-thymidine uptake by the IL-6-dependent cells is measured in the presence of an IL-6 inhibitor. Alternatively, IL-6 receptor-expressing U266 cells are cultured, and ¹²¹I-labeled IL-6 and an IL-6 inhibitor are added to the culture at the same time; and then ¹²⁵I-labeled IL-6 bound to the IL-6 receptor-expressing cells is quantified. In addition to the IL-6 inhibitor group, a negative control group that does not contain an IL-6 inhibitor is included in the assay system described above. The activity of the IL-6 inhibitor to inhibit IL-6 can be evaluated by comparing the results of both groups. The activity of the IL-6 inhibitor in inhibiting IL-6 signaling in prostate cancer cell lines can also be assessed by quantifying the STAT3 phosphorylation downstream of the IL-6 receptor signal pathway. When the STAT3 phosphorylation is suppressed by adding an IL-6 inhibitor, the IL-6 inhibitor is considered to inhibit IL-6 signaling and to suppress prostate cancer growth. STAT3 phosphorylation can be determined by known methods, and also by methods described in the Examples.

As shown below in the Examples, administration of an anti-IL-6 receptor antibody was found to suppress prostate cancer growth. This finding suggests that IL-6 inhibitors such as anti-IL-6 receptor antibodies are useful as agents for treating prostate cancer.

Subjects to be administered with the prostate cancer therapeutic agents of the present invention are mammals. The mammals are preferably humans.

The prostate cancer therapeutic agents can be administered as pharmaceuticals, and may be administered systemically or locally via oral or parenteral administration. For example, intravenous injections such as drip infusions, intramuscular injections, intraperitoneal injections, subcutaneous injections, suppositories, enemas, oral enteric tablets, or the like can be selected. Appropriate administration methods can be selected depending on a patient's age and symptoms. The effective dose per administration is selected from the range of 0.01 to 100 mg/kg body weight. Alternatively, the dose may be selected from the range of 1 to 1000 mg/patient, preferably from the range of 5 to 50 mg/patient. A preferred dose and administration method are as follows: For example, when an anti-IL-6 receptor antibody is used, the effective dose is an amount such that free antibody is present in the blood. Specifically, a dose of 0.5 to 40 mg/kg body weight/month (four weeks), preferably 1 to 20 mg/kg body weight/month is administered via an intravenous injection such as a drip infusion, subcutaneous injection or such, once to several times a month, for example, twice a week, once a week, once every two weeks, or once every four weeks. The administration schedule may be adjusted by, for example, extending the administration interval of twice a week or once a week to once every two weeks, once every three weeks, or once every four weeks, while monitoring the condition after administration and changes in the blood test values.

The agents for treating prostate cancer may contain pharmaceutically acceptable carriers, such as preservatives and stabilizers. "Pharmaceutically acceptable carriers" refer to materials that can be co-administered with an above-described agent; and may or may not-themselves produce the above-described effect of suppressing prostate cancer growth. Alternatively, the carriers may be materials that do not have the effect of suppressing prostate cancer growth, but that produce an additive or synergistic stabilizing effect when used in combination with an IL-6 inhibitor.

Such pharmaceutically acceptable materials include, for example, sterile water, physiological saline, stabilizers, excipients, buffers, preservatives, detergents, chelating agents (EDTA and such), and binders.

In the present invention, detergents include non-ionic detergents, and typical examples of such include sorbitan fatty acid esters such as sorbitan monocaprylate, sorbitan monolaurate, and sorbitan monopalmitate; glycerin fatty acid esters such as glycerin monocaprylate, glycerin monomyristate and glycerin monostearate; polyglycerin fatty acid esters such as decaglyceryl monostearate, decaglyceryl distearate, and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol, polyoxyethylene polyoxypropylene propyl ether, and polyoxyethylene polyoxypropylene cetyl ether; polyoxyethylene alkyl phenyl ethers such as polyoxyethylene nonylphenyl ether; polyoxyethylene hardened castor oils such as polyoxyethylene castor oil and polyoxyethylene hardened castor oil (polyoxyethylene hydrogenated castor oil); polyoxyethylene beeswax derivatives such as polyoxyethylene sorbit beeswax; polyoxyethylene lanolin derivatives such as polyoxyethylene lanolin; and polyoxyethylene fatty acid amides and such with an HLB of six to 18, such as polyoxyethylene stearic acid amide.

Detergents also include anionic detergents, and typical examples of such include, for example, alkylsulfates having an alkyl group with ten to 18 carbon atoms, such as sodium cetylsulfate, sodium laurylsulfate, and sodium oleylsulfate; polyoxyethylene alkyl ether sulfates in which the alkyl group has ten to 18 carbon atoms and the average molar number of added ethylene oxide is 2 to 4, such as sodium polyoxyethylene lauryl sulfate; alkyl sulfosuccinate ester salts having an alkyl group with eight to 18 carbon atoms, such as sodium lauryl sulfosuccinate ester; natural detergents, for example, lecithin; glycerophospholipids; sphingo-phospholipids such as sphingomyelin; and sucrose fatty acid esters in which the fatty acids have 12 to 18 carbon atoms.

One, two or more of the detergents described above can be combined and added to the agents of the present invention. Detergents that are preferably used in the preparations of the present invention include polyoxyethylene sorbitan fatty acid esters, such as polysorbates 20, 40, 60, and 80. Polysorbates 20 and 80 are particularly preferred. Polyoxyethylene polyoxypropylene glycols, such as poloxamer (Pluronic F-68(R) and such), are also preferred.

The amount of detergent added varies depending on the type of detergent used. When polysorbate 20 or 80 is used, the amount is in general in the range of 0.001 to 100 mg/ml, preferably in the range of 0.003 to 50 mg/ml, more preferably in the range of 0.005 to 2 mg/ml.

In the present invention, buffers include phosphate, citrate buffer, acetic acid, malic acid, tartaric acid, succinic acid, lactic acid, potassium phosphate, gluconic acid, capric acid, deoxycholic acid, salicylic acid, triethanolamine, fumaric acid, and other organic acids; and carbonic acid buffer, Tris buffer, histidine buffer, and imidazole buffer.

Liquid preparations may be formulated by dissolving the agents in aqueous buffers known in the field of liquid preparations. The buffer concentration is in general in the range of 1 to 500 mM, preferably in the range of 5 to 100 mM, more preferably in the range of 10 to 20 mM.

The agents of the present invention may also comprise other low-molecular-weight polypeptides; proteins such as serum albumin, gelatin, and immunoglobulin; amino acids; sugars and carbohydrates such as polysaccharides and monosaccharides, sugar alcohols, and such.

Herein, amino acids include basic amino acids, for example, arginine, lysine, histidine, and ornithine, and inorganic salts of these amino acids (preferably hydrochloride salts, and phosphate salts, namely phosphate amino acids). When free amino acids are used, the pH is adjusted to a preferred value by adding appropriate physiologically acceptable buffering substances, for example, inorganic acids, and in particular hydrochloric acid, phosphoric acid, sulfuric acid, acetic acid, and formic acid, and salts thereof. In this case, the use of phosphate is particularly beneficial because it gives quite stable freeze-dried products. Phosphate is particularly advantageous when preparations do not substantially contain organic acids, such as malic acid, tartaric acid, citric acid, succinic acid, and fumaric acid, or do not contain corresponding anions (malate ion, tartrate ion, citrate ion, succinate ion, fumarate ion, and such). Preferred amino acids are arginine, lysine, histidine, and ornithine. Acidic amino acids can also be used, for example, glutamic acid and aspartic acid, and salts thereof (preferably sodium salts); neutral amino acids, for example, isoleucine, leucine, glycine, serine, threonine, valine, methionine, cysteine, and alanine; and aromatic amino acids, for example, phenylalanine, tyrosine, tryptophan, and its derivative, N-acetyl tryptophan.

Herein, sugars and carbohydrates such as polysaccharides and monosaccharides include, for example, dextran, glucose, fructose, lactose, xylose, mannose, maltose, sucrose, trehalose, and raffinose.

Herein, sugar alcohols include, for example, mannitol, sorbitol, and inositol.

When the agents of the present invention are prepared as aqueous solutions for injection, the agents may be mixed with, for example, physiological saline, and/or isotonic solution containing glucose or other auxiliary agents (such as D-sorbitol, D-mannose, D-mannitol, and sodium chloride). The aqueous solutions may be used in combination with appropriate solubilizing agents such as alcohols (ethanol and such), polyalcohols (propylene glycol, PEG, and such), or non-ionic detergents (polysorbate 80 and HCO-50).

The agents may further comprise, if required, diluents, solubilizers, pH adjusters, soothing agents, sulfur-containing reducing agents, antioxidants, and such.

Herein, the sulfur-containing reducing agents include, for example, compounds comprising sulfhydryl groups, such as N-acetylcysteine, N-acetylhomocysteine, thioctic acid, thiodiglycol, thioethanolamine, thioglycerol, thiosorbitol, thioglycolic acid and salts thereof, sodium thiosulfate, glutathione, and thioalkanoic acids having one to seven carbon atoms.

Moreover, the antioxidants in the present invention include, for example, erythorbic acid, dibutylhydroxy toluene, butylhydroxy anisole, α-tocopherol, tocopherol acetate, L-ascorbic acid and salts thereof, L-ascorbic acid palmitate, L-ascorbic acid stearate, sodium hydrogen sulfite, sodium sulfite, triamyl gallate, propyl gallate, and chelating agents such as disodium ethylenediamine tetraacetate (EDTA), sodium pyrophosphate, and sodium metaphosphate.

If required, the agents may be encapsulated in microcapsules (microcapsules of hydroxymethylcellulose, gelatin, poly[methylmethacrylic acid] or such) or prepared as colloidal drug delivery systems (liposome, albumin microspheres, microemulsion, nano-particles, nano-capsules, and such)(see "Remington's Pharmaceutical Science 16th edition", Oslo Ed., 1980, and the like). Furthermore, methods for preparing agents as sustained-release agents are also known, and are applicable to the present invention *(*Langer et al., J. Biomed. Mater. Res. 1981, 15: 167-277; Langer, Chem. Tech. 1982, 12: 98-105; U.S. Patent No. 3,773,919; European Patent Application No. (EP) 58,481; Sidman et al., Biopolymers 1983, 22: 547-556; and EP 133,988).

Pharmaceutically acceptable carriers used are appropriately selected from those described above or combined depending on the type of dosage form, but are not limited thereto.

The present disclosure relates to methods for treating prostate cancer, which comprise the step of administering an IL-6 inhibitor to subjects who have developed prostate cancer.

Herein, the "subject" refers to the organisms or organism body parts to be administered with an agent of the present invention for treating prostate cancer. The organisms include animals (for example, human, domestic animal species, and wild animals) but are not particularly limited.

The "organism body parts" are not particularly limited, but preferably include the prostate gland, peripheral parts of the prostate gland, or metastatic parts.

Herein, "administration" includes oral and parenteral administration. Oral administration includes, for example, administration of oral agents. Such oral agents include, for example, granules, powders, tablets, capsules, solutions, emulsions, and suspensions.

Parenteral administration includes, for example, administration of injections. Such injections include, for example, intravenous injection such as infusion, subcutaneous injections, intramuscular injections, and intraperitoneal injection. Meanwhile, the effects of the methods of the present invention can be achieved by introducing genes comprising oligonucleotides to be administered to living bodies using gene therapy techniques. Alternatively, the agents of the present invention may be administered locally to intended areas of treatment. For example, the agents can be administered by local injection during surgery, use of catheters, or targeted gene delivery of DNAs encoding peptides of the present invention. The agents of the present invention may be administered at the same time with known therapeutic methods for prostate cancer, for example, prostatectomy, radiotherapy, hormonal therapy, chemotherapy, and such, or at different times.

### [Examples]

Hereinbelow, the present invention will be specifically described with reference to the Examples, but it is not to be construed as being limited thereto.

### [Example 1] Confirmation of IL-6 receptor expression in human prostate cancer cell lines

Whether human prostate cancer cell lines express IL-6 receptor was examined by Western blotting as described below. The human prostate cancer cell lines used were PC3, DU145, and JCA-1.

The cell lines described above were cultured in RPMI 1640 medium (Invitrogen, Groningen, The Netherlands) supplemented with 10% fetal bovine serum (FBS) and streptomycin, and then cooled on ice and washed twice with phosphate-buffered saline (PBS). The cells were harvested and lysed with 200 µl of RIPA buffer (20 mM Tris-HCl (pH 7.4), 150 mM NaCl, 2 mM ethylenediaminetetraacetic acid, 1% NP-40, 1% sodium deoxycholate, 0.1% sodium dodecyl sulfate [SDS], 50 mM NaF, 1mM sodium orthvanadate, 1 mM phenylmethylsulfonyl fluoride, 10 µg/ml aprotinin, and 10 µg/ml leupeptin). The protein concentration of the supernatants was determined by a dye binding method according to the manufacturer's instructions (BioRad Laboratories, Hercules California). The proteins thus obtained were transferred onto a nitrocellulose membrane (BioRad Laboratories) using SDS-polyacrylamide gel electrophoresis. Non-specific adsorption was suppressed using Tris-buffered physiological saline containing 5% skimmed milk, and the membrane was reacted with a 500-times diluted rabbit anti-human IL-6 receptor antibody (Santa Cruz Biotechnology). Immunoreactive bands were obtained using the Amplified Alkaline Phosphatase System according to the manufacturer's instructions (BioRad Laboratories).

The result showed that human prostate cancer cells JCA-1, PC3, and DU145 expressed IL-6 receptor (Fig. 1).

### [Example 2] Confirmation of IL-6 production in human prostate cancer cell lines

Whether IL-6 is produced in human prostate cancer cell lines was examined by the methods described below.

Specifically, the cell lines described in Example 1 were cultured at 5 x 10⁴ cells/well in 24-well plates for 48 hours using the medium described in Example 1, and the IL-6 concentration in the supernatants was determined by ELISA.

The result showed that IL-6 was produced in human prostate cancer cells JCA-1, PC3, and DU145 (Fig. 2).

### [Example 3] Confirmation of the in vitro antitumor effect of hPM1

Whether hPM1 has an *in vitro* antitumor effect was examined by the methods described below.

The cell lines described in Example 1 were cultured at 5 x 10³ cells/well in 96-well plates for 24 hours using RPM1 1640 medium (Invitrogen, Groningen, The Netherlands) supplemented with 5% fetal bovine serum (FBS) and streptomycin, and then, humanized PM-1 antibody (hPM1) was added at concentrations of 30, 100, or 300 µl/ml. After 24 and 48 hours, the antitumor effect was assayed by MTT assay. hPM1 (Hirata T et al., Leuk Res. 2003; 27(4):343-9, Sato K et al., Cancer Res. 1993; 53(4):851-6) was provided by Chugai Pharmaceutical Co. Ltd.

The result showed that hPM1 suppressed cell growth in a time- and concentration-dependent manner (Fig. 3).

### [Example 4] Confirmation of the IL-6 receptor-mediated in vitro effect of hPM1

Whether the hPM1 effect is exerted through IL-6 receptor was examined by the methods described below.

The present inventors focused on STAT3 downstream of the IL-6 receptor signal pathway. DU145 was cultured for 24 hours in the medium described above, and then 100 µg/ml hPM1 was added thereto. After 60 minutes, the STAT3 phosphorylation was compared with that of a control by Western blotting as described above. Then, after 24 hours of culture, the cells were stimulated with 10 ng/ml IL-6 in the presence or absence of 100 µg/ml hPM1, and then, the STAT3 phosphorylation after 30 minutes and 60 minutes was compared by Western blotting. The primary antibody used was a 1000-times diluted rabbit anti-human pSTAT3 antibody (Cell Signaling Technology).

The result showed that the STAT3 phosphorylation was suppressed in the prostate cancer cell lines 60 minutes after hPM1 administration. The result also showed that hPM1 suppressed the IL-6 stimulation-enhanced STAT3 phosphorylation (Fig. 4).

### [Example 5] Confirmation of the in vivo antitumor effect of hPM1

Whether hPM1 has an antitumor effect *in vivo* was confirmed by the methods described below.

DU145 cells (10⁷ cells/body) were subcutaneously transplanted into SCID mice to produce a subcutaneous tumor model. hPM1 administration (200 µg/body, every three days) was started five days after the tumor graft was confirmed. The tumor volume and mouse weight were measured over time to assess the *in vivo* antitumor effect of hPM1.

The result showed that hPM1 administration to the DU145-transplanted SCID mice significantly suppressed the tumor growth and weight loss (Fig. 5). The suppression of weight loss in the tumor model suggests that IL-6 inhibitors are also effective for treating cachexia that accompanies prostate cancer.

### Industrial Applicability

Hormonal therapy is the only therapeutic method available for treating advanced prostate cancer. However, many advanced prostate cancer patients acquire hormone resistance several years after starting hormonal therapy, and they struggle with the treatment. The prostate cancer therapeutic agents of the present disclosure, which comprise an IL-6 inhibitor as an active ingredient, are expected to be an effective alternative to hormonal therapy and to be therapeutically effective even for prostate cancer that has acquired resistance to hormonal therapy. The prostate cancer therapeutic agents of the present disclosure are expected to have an effect of suppressing post-surgery prostate cancer recurrence. The prostate cancer therapeutic agents are considered to be effective for treating cachexia that accompanies prostate cancer.

## Claims

1. A pharmaceutical composition comprising an antibody that recognizes an IL-6 receptor and blocks IL-6-mediated signal transduction as an active ingredient for use in treating cachexia that accompanies prostate cancer.

2. The pharmaceutical composition of claim 1 for use in treating cachexia that accompanies prostate cancer, wherein the antibody is a monoclonal antibody.

3. The pharmaceutical composition of claim 1 for use in treating cachexia that accompanies prostate cancer, wherein the antibody recognizes a human IL-6 receptor.

4. The pharmaceutical composition of claim 1 for use in treating cachexia that accompanies prostate cancer, wherein the antibody is a recombinant antibody.

5. The pharmaceutical composition of claim 4 for use in treating cachexia that accompanies prostate cancer, wherein the antibody is a chimeric, humanized, or human antibody.

6. The pharmaceutical composition of any one of claims 1 to 5, for the use according to claim 1 wherein the prostate cancer is hormone-refractory prostate cancer.

7. Use of an antibody that recognizes an IL-6 receptor and blocks IL-6-mediated signal transduction for the preparation of a pharmaceutical composition for treating cachexia that accompanies prostate cancer_{.}

8. The use of claim 7, wherein the antibody is a monoclonal antibody.

9. The use of claim 7, wherein the antibody recognizes a human IL-6 receptor.

10. The use of claim 7, wherein the antibody is a recombinant antibody.

11. The use of claim 10, wherein the antibody is a chimeric, humanized, or human antibody.

12. The use of any one of claims 7 to 11, wherein the prostate cancer is hormone-refractory prostate cancer.

## Patentansprüche

1. Arzneimittel umfassend einen Antiköper, der einen IL-6-Rezeptor erkennt und IL-6-vermittelte Signalübertragung blockiert, als Wirkstoff zur Verwendung bei der Behandlung von Prostatakrebs-begleitender Kachexie.

2. Arzneimittel nach Anspruch 1 zur Verwendung bei der Behandlung von Prostatakrebs-begleitender Kachexie, wobei der Antikörper ein monoclonaler Antikörper ist.

3. Arzneimittel nach Anspruch 1 zur Verwendung bei der Behandlung von Prostatakrebs-begleitender Kachexie, wobei der Antikörper einen menschlichen IL-6-Rezeptor erkennt.

4. Arzneimittel nach Anspruch 1 zur Verwendung bei der Behandlung von Prostatakrebs-begleitender Kachexie, wobei der Antikörper ein rekombinanter Antiköper ist.

5. Arzneimittel nach Anspruch 4 zur Verwendung bei der Behandlung von Prostatakrebs-begleitender Kachexie, wobei der Antikörper ein chimärer, humanisierter oder menschlicher Antikörper ist.

6. Arzneimittel nach einem der Ansprüche 1 bis 5 zur Verwendung nach Anspruch 1, wobei der Prostatakrebs hormonresistenter Prostatakrebs ist.

7. Verwendung eines Antikörpers, der einen IL-6-Rezeptor erkennt und IL-6-vermittelte Signalübertragung blockiert, zur Herstellung eines Arzneimittels zur Behandlung von Prostatakrebs-begleitetender Kachexie.

8. Verwendung nach Anspruch 7, wobei der Antikörper ein monoclonaler Antikörper ist.

9. Verwendung nach Anspruch 7, wobei der Antikörper einen menschlichen IL-6-Rezeptor erkennt.

10. Verwendung nach Anspruch 7, wobei der Antikörper ein rekombinanter Antiköper ist.

11. Verwendung nach Anspruch 10, wobei der Antikörper ein chimärer, humanisierter oder menschlicher Antikörper ist.

12. Verwendung nach einem der Ansprüche 7 bis 11, wobei der Prostatakrebs hormonresistenter Prostatakrebs ist.

## Revendications

1. Composition pharmaceutique comprenant un anticorps qui reconnaît un récepteur d'IL-6 et bloque la transduction du signal médiée par l'IL-6 à titre de principe actif pour une utilisation pour traiter la cachexie qui accompagne le cancer de la prostate.

2. Composition pharmaceutique selon la revendication 1 pour une utilisation pour traiter la cachexie qui accompagne le cancer de la prostate, dans laquelle l'anticorps est un anticorps monoclonal.

3. Composition pharmaceutique selon la revendication 1 pour une utilisation pour traiter la cachexie qui accompagne le cancer de la prostate, dans laquelle l'anticorps reconnaît un récepteur d'IL-6 humain.

4. Composition pharmaceutique selon la revendication 1 pour une utilisation pour traiter la cachexie qui accompagne le cancer de la prostate, dans laquelle l'anticorps est un anticorps recombinant.

5. Composition pharmaceutique selon la revendication 4 pour une utilisation pour traiter la cachexie qui accompagne le cancer de la prostate, dans laquelle l'anticorps est un anticorps chimérique, humanisé, ou humain.

6. Composition pharmaceutique selon l'une quelconque des revendications 1 à 5, pour une utilisation selon la revendication 1, dans laquelle le cancer de la prostate est un cancer de la prostate réfractaire aux hormones.

7. Utilisation d'un anticorps qui reconnaît un récepteur d'IL-6 et bloque la transduction du signal médiée par l'IL-6 pour la préparation d'une composition pharmaceutique pour traiter la cachexie qui accompagne le cancer de la prostate.

8. Utilisation selon la revendication 7, dans laquelle l'anticorps est un anticorps monoclonal.

9. Utilisation selon la revendication 7, dans laquelle l'anticorps reconnaît un récepteur d'IL-6 humain.

10. Utilisation selon la revendication 7, dans laquelle l'anticorps est un anticorps recombinant.

11. Utilisation selon la revendication 10, dans laquelle l'anticorps est un anticorps chimérique, humanisé, ou humain.

12. Utilisation selon l'une quelconque des revendications 7 à 11, dans laquelle le cancer de la prostate est un cancer de la prostate réfractaire aux hormones.
